(19) Europäisches Patentamt
European Patent Office
Office européen des brevets

(11) EP 4 302 779 A1

(12) EUROPEAN PATENT APPLICATION
published in accordance with Art. 153(4) EPC

(43) Date of publication:
**10.01.2024 Bulletin 2024/02**

(21) Application number: **22763008.4**

(22) Date of filing: **18.02.2022**

(51) International Patent Classification (IPC):
***A61K 45/00*** (2006.01) ***A61P 35/00*** (2006.01)
***A61P 43/00*** (2006.01) ***A61K 31/335*** (2006.01)
***A61K 31/40*** (2006.01) ***A61K 31/4025*** (2006.01)
***A61K 31/454*** (2006.01) ***A61K 31/485*** (2006.01)
***A61K 31/496*** (2006.01)

(52) Cooperative Patent Classification (CPC):
**A61K 31/335; A61K 31/337; A61K 31/40; A61K 31/4025; A61K 31/454; A61K 31/485; A61K 31/496; A61K 45/00; A61K 45/06; A61P 35/00; A61P 43/00**

(86) International application number:
**PCT/JP2022/006631**

(87) International publication number:
**WO 2022/185952 (09.09.2022 Gazette 2022/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **04.03.2021 JP 2021034337**

(71) Applicant: **Socium Inc.**
**Tokyo, 103-0026 (JP)**

(72) Inventors:
- **HORIMOTO, Katsuhisa**
  **Tokyo 103-0026 (JP)**
- **KITAZAWA, Masashi**
  **Tokyo 103-0026 (JP)**
- **KIBOKU, Takayuki**
  **Tokyo 103-0026 (JP)**
- **MIZOKAMI, Atsushi**
  **Kanazawa-shi, Ishikawa 920-1164 (JP)**
- **IZUMI, Kouji**
  **Kanazawa-shi, Ishikawa 920-1164 (JP)**
- **SHIMADA, Takashi**
  **Kanazawa-shi, Ishikawa 920-1164 (JP)**

(74) Representative: **MacLean, Martin Robert et al**
**Mathys & Squire**
**The Shard**
**32 London Bridge Street**
**London SE1 9SG (GB)**

# (54) CANCER THERAPEUTIC AGENT

(57) Provided is a therapeutic agent for cellular drug resistance to a microtubule inhibitor. The therapeutic agent contains a κ opioid receptor agonist.

EP 4 302 779 A1

Fig. 12

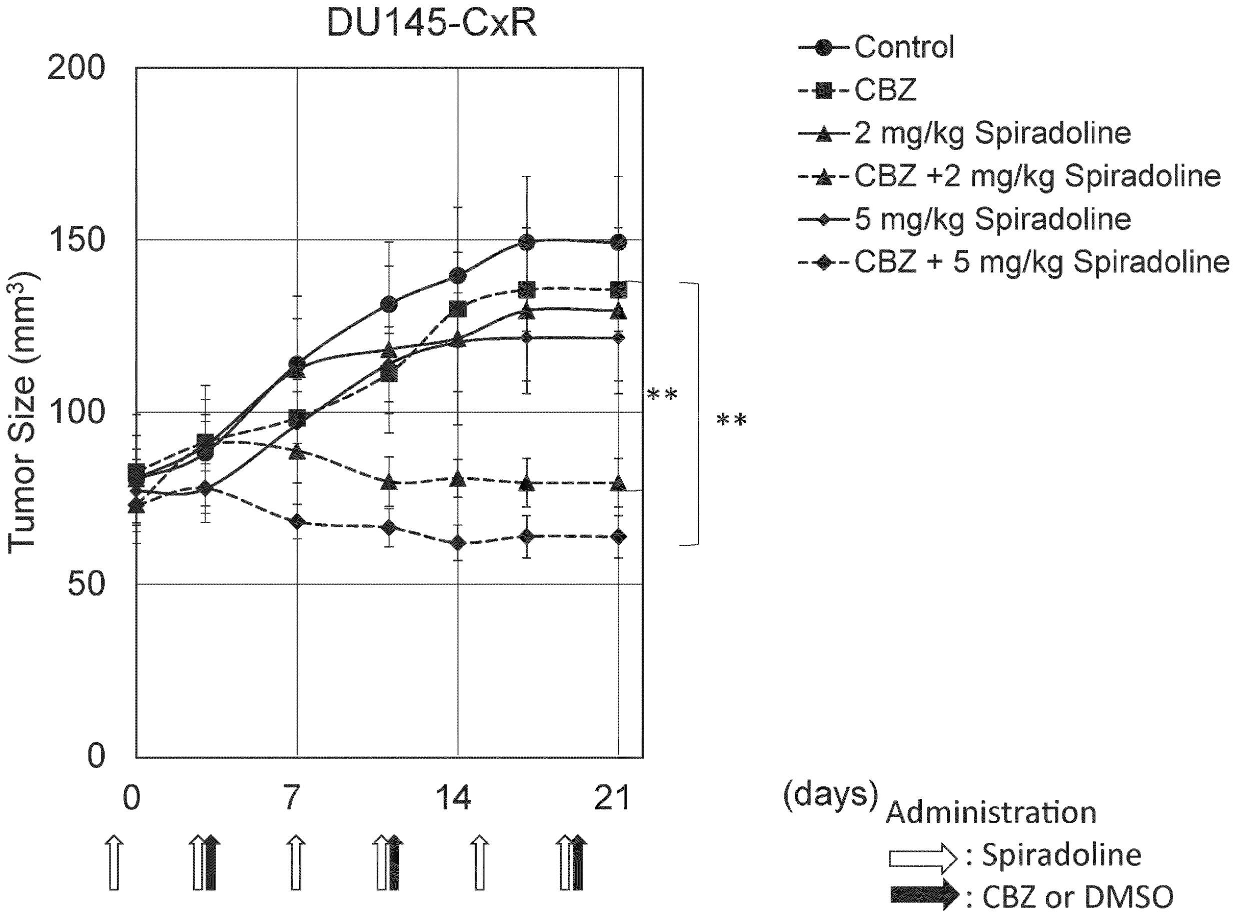
DU145-CxR
Tumor Size ($mm^3$)
200
150
100
50
0
0
7
14
21
(days)
**
**
Control
CBZ
2 mg/kg Spiradoline
CBZ +2 mg/kg Spiradoline
5 mg/kg Spiradoline
CBZ + 5 mg/kg Spiradoline
Administration
: Spiradoline
: CBZ or DMSO

## Description

### Technical Field

The present invention relates to a therapeutic agent for cellular drug resistance to a microtubule inhibitor and a pharmaceutical composition for cancer treatment.

### Background Art

Anticancer drugs used in cancer chemotherapy include taxane anticancer drugs. The taxane anticancer drugs include paclitaxel and docetaxel. When paclitaxel and docetaxel are administered to a cancer patient, the cancer cells of the patient may express drug resistance, and the patient may develop resistance to the treatment. This tends to reduce the therapeutic efficacy. The taxane anticancer drugs also include cabazitaxel. For example, cabazitaxel is efficacious for prostate cancer patients with a treatment history including docetaxel. Cabazitaxel, however, may also cause cancer cells to express drug resistance, and patients may develop treatment resistance. This tends to reduce the therapeutic efficacy (for example, see Patent Document 1).

Citation List

Patent Document

Patent Document 1: JP 6735484 B

### Summary of Invention

Technical Problem

There is a demand for a means to improve the therapeutic efficacy of anticancer drugs against any cancer including but not limited to prostate cancer. Therefore, an object of the present invention is providing a means to improve the therapeutic efficacy of an anticancer drug. Solution to Problem

According to an aspect of the present invention, a therapeutic agent for cellular drug resistance to a microtubule inhibitor is provided, and the therapeutic agent contains a κ opioid receptor agonist.

The aforementioned therapeutic agent for cellular drug resistance to a microtubule inhibitor may contain the κ opioid receptor agonist as an active component.

The aforementioned therapeutic agent for cellular drug resistance to a microtubule inhibitor may contain the κ opioid receptor agonist in an effective amount for treating cellular drug resistance to a microtubule inhibitor.

In the aforementioned therapeutic agent for cellular drug resistance to a microtubule inhibitor, the κ opioid receptor agonist may be at least one selected from the group consisting of spiradoline, nalfurafine, ICI-199,441, LPK-26, BRL-52537, GR-89696, and salts thereof.

In the aforementioned therapeutic agent for cellular drug resistance to a microtubule inhibitor, the κ opioid receptor agonist may be at least one of spiradoline and salts thereof.

In the aforementioned therapeutic agent for cellular drug resistance to a microtubule inhibitor, the salt may be a physiologically acceptable salt.

In the aforementioned therapeutic agent for cellular drug resistance to a microtubule inhibitor, the microtubule inhibitor may be a taxane anticancer drug.

In the aforementioned therapeutic agent for cellular drug resistance to a microtubule inhibitor, the taxane anticancer drug may be at least one selected from the group consisting of cabazitaxel, paclitaxel, and docetaxel.

In the aforementioned therapeutic agent for cellular drug resistance to a microtubule inhibitor, the taxane anticancer drug may be cabazitaxel.

In the aforementioned therapeutic agent for cellular drug resistance to a microtubule inhibitor, the cancer may be at least one selected from the group consisting of prostate cancer, bladder cancer, breast cancer, stomach cancer, and uterine cancer.

In the aforementioned therapeutic agent for cellular drug resistance to a microtubule inhibitor, the cancer may be genitourinary cancer. The genitourinary cancer may be at least one selected from prostate cancer, bladder cancer, kidney cancer, renal pelvis and ureter cancer, testicular cancer, and penile cancer.

In the aforementioned therapeutic agent for cellular drug resistance to a microtubule inhibitor, the cancer may be prostate cancer. The cancer may be breast cancer. The cancer may be uterine cancer.

The aforementioned therapeutic agent for cellular drug resistance to a microtubule inhibitor may be administered to a cancer patient. The patient may be a human or a non-human animal.

According to an aspect of the present invention, use of a κ opioid receptor agonist for producing a therapeutic agent for cellular drug resistance to a microtubule inhibitor is provided.

In the aforementioned use, the therapeutic agent for cellular drug resistance to a microtubule inhibitor may contain the κ opioid receptor agonist as an active component.

In the aforementioned use, the therapeutic agent for cellular drug resistance to a microtubule inhibitor may contain the κ opioid receptor agonist in an effective amount for treating cellular drug resistance to a microtubule inhibitor.

In the aforementioned use, the κ opioid receptor agonist may be at least one selected from the group consisting of spiradoline, nalfurafine, ICI-199,441, LPK-26, BRL-52537, GR-89696, and salts thereof.

In the aforementioned use, the κ opioid receptor agonist may be at least one of spiradoline and salts thereof.

In the aforementioned use, the salt may be a physiologically acceptable salt.

In the aforementioned use, the microtubule inhibitor may be a taxane anticancer drug.

In the aforementioned use, the taxane anticancer drug may be at least one selected from the group consisting of cabazitaxel, paclitaxel, and docetaxel.

In the aforementioned use, the taxane anticancer drug may be cabazitaxel.

In the aforementioned use, the cancer may be at least one selected from the group consisting of prostate cancer, bladder cancer, breast cancer, stomach cancer, and uterine cancer.

In the aforementioned use, the cancer may be genitourinary cancer. The genitourinary cancer may be at least one selected from prostate cancer, bladder cancer, kidney cancer, renal pelvis and ureter cancer, testicular cancer, and penile cancer.

In the aforementioned use, the cancer may be prostate cancer. The cancer may be breast cancer. The cancer may be uterine cancer.

In the aforementioned use, the therapeutic agent for cellular drug resistance to a microtubule inhibitor may be administered to a cancer patient. The patient may be a human or a non-human animal.

According to an aspect of the present invention, a method for treating cellular drug resistance to a microtubule inhibitor is provided, and the method includes administering a κ opioid receptor agonist to a patient.

In the aforementioned method for treating cellular drug resistance to a microtubule inhibitor, the κ opioid receptor agonist may be administered as an active component to a patient.

In the aforementioned method for treating cellular drug resistance to a microtubule inhibitor, the κ opioid receptor agonist may be administered to a patient in an effective amount for treating cellular drug resistance to a microtubule inhibitor.

In the aforementioned method for treating cellular drug resistance to a microtubule inhibitor, the κ opioid receptor agonist may be at least one selected from the group consisting of spiradoline, nalfurafine, ICI-199,441, LPK-26, BRL-52537, GR-89696, and salts thereof.

In the aforementioned method for treating cellular drug resistance to a microtubule inhibitor, the κ opioid receptor agonist may be at least one of spiradoline and salts thereof.

In the aforementioned method for treating cellular drug resistance to a microtubule inhibitor, the salt may be a physiologically acceptable salt.

In the aforementioned method for treating cellular drug resistance to a microtubule inhibitor, the microtubule inhibitor may be a taxane anticancer drug.

In the aforementioned method for treating cellular drug resistance to a microtubule inhibitor, the taxane anticancer drug may be at least one selected from the group consisting of cabazitaxel, paclitaxel, and docetaxel.

In the aforementioned method for treating cellular drug resistance to a microtubule inhibitor, the taxane anticancer drug may be cabazitaxel.

In the aforementioned method for treating cellular drug resistance to a microtubule inhibitor, the cancer may be at least one selected from the group consisting of prostate cancer, bladder cancer, breast cancer, stomach cancer, and uterine cancer.

In the aforementioned method for treating cellular drug resistance to a microtubule inhibitor, the cancer may be genitourinary cancer. The genitourinary cancer may be at least one selected from prostate cancer, bladder cancer, kidney cancer, renal pelvis and ureter cancer, testicular cancer, and penile cancer.

In the aforementioned method for treating cellular drug resistance to a microtubule inhibitor, the cancer may be prostate cancer. The cancer may be breast cancer. The cancer may be uterine cancer.

In the aforementioned method for treating cellular drug resistance to a microtubule inhibitor, the patient may be a cancer patient. The patient may be a human or a non-human animal.

According to an aspect of the present invention, a pharmaceutical composition for cancer treatment is provided, and the pharmaceutical composition contains a κ opioid receptor agonist and a microtubule inhibitor.

The aforementioned pharmaceutical composition for cancer treatment may contain the κ opioid receptor agonist as an active component to treat cellular drug resistance to the microtubule inhibitor.

The aforementioned pharmaceutical composition for cancer treatment may contain the κ opioid receptor agonist in an effective amount for treating cellular drug resistance to the microtubule inhibitor.

In the aforementioned pharmaceutical composition for cancer treatment, the κ opioid receptor agonist may be at least one selected from the group consisting of spiradoline, nalfurafine, ICI-199,441, LPK-26, BRL-52537, GR-89696, and salts thereof.

In the aforementioned pharmaceutical composition for cancer treatment, the κ opioid receptor agonist may be at least one of spiradoline and salts thereof.

In the aforementioned pharmaceutical composition for cancer treatment, the salt may be a physiologically acceptable salt.

In the aforementioned pharmaceutical composition for cancer treatment, the microtubule inhibitor may be a taxane anticancer drug.

In the aforementioned pharmaceutical composition for cancer treatment, the taxane anticancer drug may be at least one selected from the group consisting of cabazitaxel, paclitaxel, and docetaxel.

In the aforementioned pharmaceutical composition for cancer treatment, the taxane anticancer drug may be cabazitaxel.

In the aforementioned pharmaceutical composition for cancer treatment, the cancer may be at least one selected from the group consisting of prostate cancer, bladder cancer, breast cancer, stomach cancer, and uterine cancer.

In the aforementioned pharmaceutical composition for cancer treatment, the cancer may be genitourinary cancer. The genitourinary cancer may be at least one selected from prostate cancer, bladder cancer, kidney cancer, renal pelvis and ureter cancer, testicular cancer, and penile cancer.

In the aforementioned pharmaceutical composition for cancer treatment, the cancer may be prostate cancer. The cancer may be breast cancer. The cancer may be uterine cancer.

According to an aspect of the present invention, use of a κ opioid receptor agonist and a microtubule inhibitor for producing a pharmaceutical composition for cancer treatment.

In the aforementioned use, the pharmaceutical composition for cancer treatment may contain the κ opioid receptor agonist as an active component to treat cellular drug resistance to the microtubule inhibitor.

In the aforementioned use, the pharmaceutical composition for cancer treatment may contain the κ opioid receptor agonist in an effective amount for treating cellular drug resistance to a microtubule inhibitor.

In the aforementioned use, the κ opioid receptor agonist may be at least one selected from the group consisting of spiradoline, nalfurafine, ICI-199,441, LPK-26, BRL-52537, GR-89696, and salts thereof.

In the aforementioned use, the κ opioid receptor agonist may be at least one of spiradoline and salts thereof.

In the use, the salt may be a physiologically acceptable salt.

In the aforementioned use, the microtubule inhibitor may be a taxane anticancer drug.

In the aforementioned use, the taxane anticancer drug may be at least one selected from the group consisting of cabazitaxel, paclitaxel, and docetaxel.

In the aforementioned use, the taxane anticancer drug may be cabazitaxel.

In the aforementioned use, the cancer may be at least one selected from the group consisting of prostate cancer, bladder cancer, breast cancer, stomach cancer, and uterine cancer.

In the aforementioned use, the cancer may be genitourinary cancer. The genitourinary cancer may be at least one selected from prostate cancer, bladder cancer, kidney cancer, renal pelvis and ureter cancer, testicular cancer, and penile cancer.

In the aforementioned use, the cancer may be prostate cancer. The cancer may be breast cancer. The cancer may be uterine cancer.

According to an aspect of the present invention, a method for treating cancer is provided, and the method includes administering a κ opioid receptor agonist and a microtubule inhibitor to a patient.

In the aforementioned method for treating cancer, the κ opioid receptor agonist may be administered to a patient as an active component to treat cellular drug resistance to the microtubule inhibitor.

In the aforementioned method for treating cancer, the κ opioid receptor agonist may be administered to a patient in an effective amount for treating cellular drug resistance to the microtubule inhibitor.

In the aforementioned method for treating cancer, the κ opioid receptor agonist may be at least one selected from the group consisting of spiradoline, nalfurafine, ICI-199,441, LPK-26, BRL-52537, GR-89696, and salts thereof.

In the aforementioned method for treating cancer, the κ opioid receptor agonist may be at least one of spiradoline and salts thereof.

In the aforementioned method for treating cancer, the salt may be a physiologically acceptable salt.

In the aforementioned method for treating cancer, the microtubule inhibitor may be a taxane anticancer drug.

In the aforementioned method for treating cancer, the taxane anticancer drug may be at least one selected from the group consisting of cabazitaxel, paclitaxel, and docetaxel.

In the aforementioned method for treating cancer, the taxane anticancer drug may be cabazitaxel.

In the aforementioned method for treating cancer, the cancer may be at least one selected from the group consisting of prostate cancer, bladder cancer, breast cancer, stomach cancer, and uterine cancer.

In the aforementioned method for treating cancer, the cancer may be genitourinary cancer. The genitourinary cancer may be at least one selected from prostate cancer, bladder cancer, kidney cancer, renal pelvis and ureter cancer, testicular cancer, and penile cancer.

In the aforementioned method for treating cancer, the cancer may be prostate cancer. The cancer may be breast cancer. The cancer may be uterine cancer.

In the aforementioned method for treating cancer, the patient may be a human or a non-human animal.

Advantageous Effects of Invention

According to the present invention, it is possible to provide a means to improve the therapeutic efficacy of an anticancer drug.

## Brief Description of Drawings

Fig. 1 is a table showing combination compounds used in Example 1.

Fig. 2 is a graph showing the relation between the concentration of cabazitaxel and the viability of DU145 cells not resistant to taxane anticancer drugs in Example 1.

Fig. 3 is a graph showing the relation between the concentration of cabazitaxel and the viability of DU145 cells resistant to docetaxel in Example 1.

Fig. 4 is a graph showing the relation between the concentration of cabazitaxel and the viability of DU145 cells resistant to cabazitaxel in Example 1.

Fig. 5 is a table showing $IC_{50}$ of cabazitaxel in Example 1.

Fig. 6 is a graph showing the relation between the concentration of cabazitaxel and the viability of PC3 cells resistant to docetaxel in Example 2.

Fig. 7 is a graph showing the relation between the concentration of cabazitaxel and the viability of PC3 cells resistant to cabazitaxel in Example 2.

Fig. 8 is a table showing $IC_{50}$ of cabazitaxel in Example 2.

Fig. 9 is a graph showing the relation between the concentration of paclitaxel and the viability of PC3 cells resistant to docetaxel in Example 3.

Fig. 10 is a graph showing the relation between the concentration of docetaxel and the viability of PC3 cells resistant to docetaxel in Example 3.

Fig. 11 is a table showing $IC_{50}$ of cabazitaxel when κ opioid receptor agonists were used in combination in Example 4.

Fig. 12 is a graph showing changes over time in tumor size of mice in Example 5.

Fig. 13 is a graph showing the relation between the concentration of paclitaxel and the viability of MCF7-TxR cells resistant to paclitaxel in Example 7.

Fig. 14 is a graph showing the relation between the concentration of docetaxel and the viability of MCF7-TxR cells resistant to paclitaxel in Example 7.

Fig. 15 is a graph showing the relation between the concentration of paclitaxel and the viability of MES-SA/Dx5 cells resistant to multiple drugs in Example 7.

Fig. 16 is a graph showing the relation between the concentration of docetaxel and the viability of MES-SA/Dx5 cells resistant to multiple drugs in Example 7.

## Description of Embodiments

A therapeutic agent for cellular drug resistance to a microtubule inhibitor according to an embodiment of the present invention contains a κ opioid receptor agonist.

A microtubule inhibitor inhibits the depolymerization of cellular microtubules to inhibit cell division. The microtubules play an important role on cell division. In cells, microtubules are polymerized until the metaphase of cell division. In the anaphase of cell division, microtubules are depolymerized in cells. The microtubule inhibitor inhibits the depolymerization of microtubules in cells and thereby inhibits cell division.

Cancer cells proliferate through repeated cell division. The microtubule inhibitor inhibits the cell division of cancer cells and is usable as an anticancer drug. The microtubule inhibitor is, for example, a taxane anticancer drug. The taxane anticancer drug has a taxane ring. Examples of the taxane anticancer drug include cabazitaxel, paclitaxel, and docetaxel. The cancer targeted by the taxane anticancer drug is, for example, genitourinary cancer. Examples of the genitourinary cancer include prostate cancer, bladder cancer, kidney cancer, renal pelvis and ureter cancer, testicular cancer, and penile cancer. The cancer targeted by the taxane anticancer drug is not limited to the genitourinary cancer and may be, for example, breast cancer, uterine cancer, stomach cancer, ovarian cancer, non-small cell lung cancer, stomach cancer, or esophagus cancer.

When a microtubule inhibitor is administered to a cancer patient, the cancer cells may exhibit drug resistance to the microtubule inhibitor to cause treatment resistance, and the microtubule inhibitor tends to have a lower therapeutic efficacy. However, a κ opioid receptor agonist treats the treatment resistance of a patient to the microtubule inhibitor.

Examples of the κ opioid receptor agonist include spiradoline, nalfurafine, ICI-199,441, LPK-26, BRL-52537, and GR-89696. The κ opioid receptor agonist may be a physiologically acceptable salt thereof.

The IUPAC name of spiradoline (CAS number: 87151-85-7) is 2-(3,4-dichlorophenyl)-N-methyl-N-[(5R,7S,8S)-7-(pyrrolidin-1-yl)-1-oxaspiro[4.5]decan-8-yl]acetamide. The chemical formula of spiradoline is $C_{22}H_{30}Cl_2N_2O_2$. Spiradoline has the following chemical structural formula.

[Chemical Formula 1]

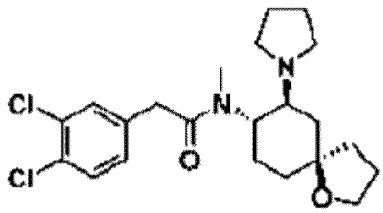

The IUPAC name of nalfurafine (CAS number: 152658-17-8) is (E)-N-[(4R,4aS,7R,7aR,12bS)-3-(cyclopropylmethyl)-4a,9-dihydroxy-1,2,4,5,6,7,7a,13-octahydro-4,12-methanobenzofuro[3,2-e]isoquinolin-7-yl]-3-(furan-3-yl)-N-methylprop-2-enamide. The chemical formula of nalfurafine is $C_{28}H_{32}N_2O_5$. Nalfurafine has the following chemical structural formula.

[Chemical Formula 2]

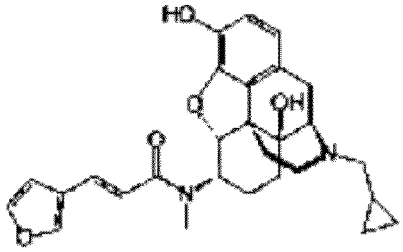

The IUPAC name of ICI-199,441 (CAS number: 115199-84-3) is 2-(3,4-dichlorophenyl)-N-methyl-N-[(1S)-1-phenyl-2-(1-pyrrolidinyl)ethyl]acetamide. The chemical formula of ICI-199, 441 is $C_{21}H_{24}Cl_2N_2O$. ICI-199,441 has the following chemical structural formula.

[Chemical Formula 3]

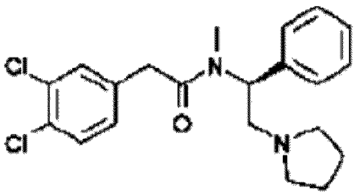

The IUPAC name of LPK-26 (CAS number: 492451-07-7) is 2-(3,4-dichlorophenyl)-N-[(2S)-1-(2,5-dihydropyrrol-1-yl)-3-methylbutan-2-yl]-N-methylacetamide. The chemical formula of LPK-26 is $C_{18}H_{24}Cl_2N_2O$. LPK-26 has the following chemical structural formula.

[Chemical Formula 4]

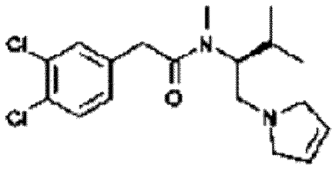

The IUPAC name of BRL-52537 (CAS number: 130497-33-5) is 2-(3,4-dichlorophenyl)-1-[(2S)-2-(pyrrolidin-1-ylmethyl)piperidin-1-yl]ethanone. The chemical formula of BRL-52537 is $C_{18}H_{24}Cl_2N_2O$. BRL-52537 has the following chemical structural formula.

[Chemical Formula 5]

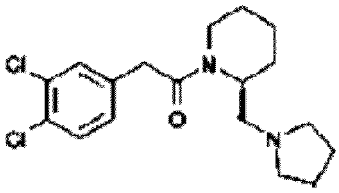

The IUPAC name of GR-89696 (CAS number: 126766-32-3) is methyl 4-[2-(3,4-dichlorophenyl)acetyl]-3-(pyrrolidin-1-ylmethyl)piperazine-1-carboxylate. The chemical formula of GR-89696 is $C_{19}H_{25}Cl_2N_3O_3$. GR-89696 has the following chemical structural formula.

[Chemical Formula 6]

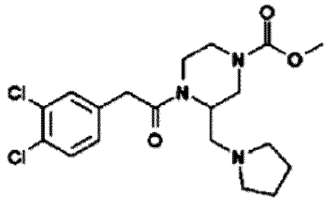

The therapeutic agent for cellular drug resistance to a microtubule inhibitor according to the embodiment of the present invention may be formulated into a pharmaceutically acceptable dosage form. For example, the therapeutic agent for cellular drug resistance to a microtubule inhibitor may be formulated into injections, solutions, suspensions, tablets, capsules, pills, granules, syrups, suppositories, inhalants, and sprays. The injections include, for example, an injection solution, a sterile powder for injection, and a concentrate for injection.

The pharmaceutical composition for cancer treatment according to the embodiment of the present invention contains a κ opioid receptor agonist and a microtubule inhibitor. The κ opioid receptor agonist and the microtubule inhibitor are as described above. The κ opioid receptor agonist and the microtubule inhibitor may be administered to a cancer patient at the same time or may be administered separately. The κ opioid receptor agonist and the microtubule inhibitor may be administered to a cancer patient at different times or on different days. The κ opioid receptor agonist and the microtubule inhibitor may be administered to a cancer patient the same number of times or different numbers of times.

The pharmaceutical composition for cancer treatment according to the embodiment of the present invention may be formulated into a pharmaceutically acceptable dosage form. For example, the pharmaceutical composition for cancer treatment may be formulated into injections, solutions, suspensions, tablets, capsules, pills, granules, syrups, suppositories, inhalants, and sprays. The injections include, for example, an injection solution, a sterile powder for injection, and a concentrate for injection. In the pharmaceutical composition for cancer treatment according to the embodiment of the present invention, the κ opioid receptor agonist and the microtubule inhibitor may be formulated separately or in combination.

The present invention has been described as above with reference to the embodiments, but the descriptions and drawings included in the disclosure should not be understood as limiting the invention. From the disclosure, various alternative embodiments, examples, and operational techniques should be apparent to a person skilled in the art. It should be understood that the invention encompasses various embodiments and the like not described in the specification.

(Example 1)

DU145 cells (ATCC) as an androgen-independent human prostate cancer cell line which did not have androgen receptor, paclitaxel- and docetaxel-resistant DU145_TxR cells, and docetaxel- and cabazitaxel-resistant DU145_CxR cells were prepared. DU145_TxR cells and DU145_CxR cells were established in accordance with Prostate, 2007, Vol. 67 (No. 9), p. 955-967.

Cells were seeded in wells of a 96-well plate at a cell number of $1 \times 10^4$ cells/well. As the culture medium, a DMEM medium containing 10% FBS was used. Twenty-four hours after cell seeding, the culture medium was replaced with a culture medium containing cabazitaxel at 0 nmol/L, 1 nmol/L, 3 nmol/L, 10 nmol/L, 30 nmol/L, or 100 nmol/L and containing any of the combination compounds shown in Fig. 1. The concentration of the combination compound was set at a concentration where the cell viability was above 90% when only the combination compound was added to cells.

Forty-eight hours after medium replacement, a reagent for colorimetric analysis to determine the number of living cells (CellTiter 96 AQueous One Solution Cell Proliferation Assay kit, Promega, G3581) was directly added to the culture medium and was reacted for 1 hour, and the absorbance was measured at 490 nm. In each condition, the cell viability was calculated where the cell viability was 100% when 0 nmol/L cabazitaxel and DMSO were added. The results are shown in Fig. 2, Fig. 3, and Fig. 4.

As shown in Fig. 2, the efficacy of cabazitaxel on the DU145 cells not resistant to the taxane anticancer drug was unchanged even when any combination compound was used. In contrast, as shown in Fig. 3 and Fig. 4, on the paclitaxel- and docetaxel-resistant DU145_TxR cells and the docetaxel- and cabazitaxel-resistant DU145_CxR cells, cabazitaxel used in combination with spiradoline (U-62066) exhibited higher efficacy than cabazitaxel with other combination compounds. The $IC_{50}$ values of cabazitaxel calculated from the graphs in Fig. 2, Fig. 3, and Fig. 4 are shown in Fig. 5. As compared with DMSO, cabazitaxel used in combination with spiradoline had markedly lower $IC_{50}$ values.

(Example 2)

PC3 cells (ATCC) as an androgen-independent human prostate cancer cell line which did not hvae androgen receptor, paclitaxel- and docetaxel-resistant PC3_TxR cells, and docetaxel- and cabazitaxel-resistant PC3_CxR cells were prepared. As combination compounds, DMSO, 0.1 μmol/L spiradoline, 1 μmol/L spiradoline, and 10 μmol/L spiradoline were prepared. Cabazitaxel was added to cells in the same conditions as in Example 1 except that these cells and combination compounds were used, and the cell viability was calculated in each condition. The results are show in Fig. 6 and Fig. 7.

As shown in Fig. 6 and Fig. 7, the efficacy of cabazitaxel on the paclitaxel- and docetaxel-resistant PC3_TxR cells and the docetaxel- and cabazitaxel-resistant PC3_CxR cells increased depending on the concentration of spiradoline. The $IC_{50}$ values of cabazitaxel calculated from the graphs in Fig. 6 and Fig. 7 are shown in Fig. 8. As compared with DMSO, cabazitaxel used in combination with spiradoline had markedly lower $IC_{50}$ values.

(Example 3)

Paclitaxel- and docetaxel-resistant PC3_TxR cells were prepared. As combination compounds, DMSO, 3 μmol/L spiradoline, and 10 μmol/L spiradoline were prepared. Paclitaxel or docetaxel was added to cells in the same conditions as in Example 1 except that these cells and combination compounds were used, and the cell viability was calculated in each condition. The results are shown in Fig. 9 and Fig. 10.

As shown in Fig. 9 and Fig. 10, the efficacies of paclitaxel and docetaxel on the paclitaxel- and docetaxel-resistant PC3_TxR cells increased depending on the concentration of spiradoline.

(Example 4)

Cabazitaxel-resistant DU145_CxR cells and PC3_CxR cells were prepared. As combination compounds, DMSO, 10 μmol/L spiradoline, nalfurafine, ICI-199,441, LPK-26, BRL-52537, and GR-89696 were prepared. Cabazitaxel was added to cells in the same conditions as in Examples 1 and 2 except that these combination compounds were used, and $IC_{50}$ values of cabazitaxel were determined from the cell viabilities in the respective conditions and were shown in Fig. 11.

As shown in Fig. 11, the efficacy of cabazitaxel on the cabazitaxel-resistant DU145_CxR cells and PC3_CxR cells increased by combination use of each of spiradoline, nalfurafine, ICI-199,441, LPK-26, BRL-52537, and GR-89696.

(Example 5)

Forty 5-week-old male mice with severe combined immunodeficiency (SCID) were purchased from CLEA Japan (Tokyo, Japan). After an acclimation period, a mixture of $3 \times 10^6$ PC3_CxR cells with 50% matrigel (Corning, New York, United States) was implanted under the dorsal skin of the SCID mice. After the mouse tumor grew enough to measure the length, 36 mice except four mice with tumor that grew insufficiently were divided into six groups such that each group had substantially the same average tumor size.

The first group was a control group; the second group was a group to which only 5 mg/kg cabazitaxel was administered; the third group was a group to which only 2 mg/kg spiradoline was administered; the fourth group was a group to which 5 mg/kg cabazitaxel and 2 mg/kg spiradoline were administered in combination; the fifth group was a group to which only 5 mg/kg spiradoline was administered; and the sixth group was a group to which 5 mg/kg cabazitaxel and 5 mg/kg spiradoline were administered in combination.

To the second, fourth, and sixth groups to which cabazitaxel was administered, a solution of cabazitaxel in 20 μL of DMSO was intraperitoneally administered once a week.

To the first, third, and fifth groups to which no cabazitaxel was administered, only 20 μL of DMSO was intraperitoneally administered at the same frequency as the administration frequency of cabazitaxel in the second, fourth, and sixth groups.

To the third, fourth, fifth, and sixth groups to which spiradoline was administered, a solution of spiradoline in 20 μL of distilled water was intraperitoneally administered twice a week.

As shown in Fig. 12, the tumor size and the mouse weight were measured simultaneously by using vernier calipers and a scale, respectively, over time. The tumor size V ($cm^3$) was calculated in accordance with the following equation where A is the major axis length (cm) of a tumor, and I is the minor axis length (cm) of the tumor.

$$V = A \times I \times I \times 0.5$$

Drugs were administered to mice on the basis of body weight over three weeks, and then, except the mouse having the smallest tumor diameter in each group, the average tumor size of five mice was calculated in each group. The results are shown in Fig. 12. The protocol was approved by the animal care and use committee of Kanazawa University Graduate School of Medicine.

As shown in Fig. 12, the tumor size of the mice to which spiradoline and cabazitaxel were administered in combination was smaller than the tumor size of the mice to which only spiradoline or cabazitaxel was administered. The tumor size of the mice to which 5 mg/kg spiradoline and cabazitaxel were administered in combination was smaller than the tumor size of the mice to which 2 mg/kg spiradoline and cabazitaxel were administered in combination.

(Example 6)

As the parent cell line, MCF-7 cells, a breast cancer cell line, were used to initiate the establishment of a paclitaxel-resistant cell line. As the culture medium, a DMEM medium containing 10% FCS was used. Paclitaxel was added to the medium at a concentration of 1 nmol/L, and the medium was replaced once every two or three days until the cells became confluent. When the cells became confluent, the cells were passaged. The concentration of paclitaxel was increased to 3 nmol/L, and the cells were cultured continuously. Similarly, the concentration of paclitaxel was then gradually increased. The cells were continuously cultured while some of the cells were cryopreserved at every passage, and a cell line that proliferated even with 12 nmol/L paclitaxel was finally established. When cells failed to proliferate at a higher concentration, the cryopreserved cells were used and cultured with paclitaxel at a concentration where the cells definitely proliferated, and the cells were passaged. The established cell line was called MCF7-TxR cells.

(Example 7)

The MCF7-TxR cells established in Example 6, MES-SA/Dx5 cells as an uterine cancer cell line resistant to multiple drugs including paclitaxel and docetaxel, MCF-7 cells as the parent cell line of the MCF7-TxR cells, and MES-SA cells as the parent cell line of the MES-SA/Dx5 cells were prepared. For the MES-SA/Dx5 cells, see Harker WG, Sikic BI. Multidrug (pleiotropic) resistance in doxorubicin-selected variants of the human sarcoma cell line MES-SA. Cancer Res. (1985) 45:4091-4096 and Chen G, Duran GE, Steger KA, Lacayo NJ, Jaffrezou JP, Dumontet C, Sikic BI. Multidrug-resistant human sarcoma cells with a mutant P-glycoprotein, altered phenotype, and resistance to cyclosporins. J Biol Chem. (1997) 272:5974-5982.

In a 384-well plate, cells were seeded at a concentration of $4 \times 10^3$ cells/well. As the culture medium for the MCF-7 cells and the MCF7-TxR cells, a DMEM medium containing 10% FBS was used. For the MES-SA cells and the MCF7-TxR cells, a RPMI1640 medium containing 10% FBS was used. Four hours after cell seeding, the culture medium was replaced with a culture medium containing 0 nmol/L, 1 nmol/L, 3 nmol/L, 10 nmol/L, 30 nmol/L, or 100 nmol/L paclitaxel or docetaxel and DMSO or 3 μmol/L spiradoline.

Forty-eight hours after medium replacement, a reagent for colorimetric analysis to determine the number of living cells (CellTiter 96 AQueous One Solution Cell Proliferation Assay kit, Promega, G3581) was directly added to the culture medium and was reacted for 1 hour, and the absorbance was measured at 490 nm. In each condition, the cell viability was calculated where the cell viability was 100% when 0 nmol/L cabazitaxel and DMSO were added.

As shown in Fig. 13, when paclitaxel was added to the culture medium of MCF-7 cells that were not drug resistant, the efficacy of paclitaxel used in combination with spiradoline was substantially the same as with DMSO. When paclitaxel was added to the culture medium of paclitaxel-resistant MCF7-TxR cells, the efficacy of paclitaxel used in combination with spiradoline was higher

than that with DMSO.

As shown in Fig. 14, when docetaxel was added to the culture medium of MCF-7 cells that were not drug resistant, the efficacy of docetaxel used in combination with spiradoline was substantially the same as with DMSO. When docetaxel was added to the culture medium of paclitaxel-resistant MCF7-TxR cells, the efficacy of docetaxel used in combination with spiradoline was higher than that with DMSO. The results suggest that the cells maintain drug resistance even when the microtubule inhibitor is changed and that the κ opioid receptor agonist lowers the resistance of the cells resistant to a plurality of microtubule inhibitors.

As shown in Fig. 15, when paclitaxel was added to the culture medium of MES-SA cells that were not drug resistant, the efficacy of paclitaxel used in combination with spiradoline was substantially the same as with DMSO. When paclitaxel was added to the culture medium of multidrug-resistant MES-SA/Dx5 cells, the efficacy of paclitaxel used in combination with spiradoline was higher than that with DMSO.

As shown in Fig. 16, when docetaxel was added to the culture medium of MES-SA cells that were not drug resistant, the efficacy of docetaxel used in combination with spiradoline was substantially the same as with DMSO. When docetaxel was added to the culture medium of multidrug-resistant MES-SA/Dx5 cells, the efficacy of docetaxel used in combination with spiradoline was higher than that with DMSO.

## Claims

1. A therapeutic agent for cellular drug resistance to a microtubule inhibitor, the therapeutic agent comprising a κ opioid receptor agonist.

2. The therapeutic agent for cellular drug resistance to a microtubule inhibitor according to claim 1, wherein the κ opioid receptor agonist is at least one selected from the group consisting of spiradoline, nalfurafine, ICI-199, 441, LPK-26, BRL-52537, GR-89696, and salts thereof.

3. The therapeutic agent for cellular drug resistance to a microtubule inhibitor according to claim 1 or 2, wherein the κ opioid receptor agonist is at least one of spiradoline and salts thereof.

4. The therapeutic agent for cellular drug resistance to a microtubule inhibitor according to any one of claims 1 to 3, wherein the microtubule inhibitor is a taxane anticancer drug.

5. The therapeutic agent for cellular drug resistance to a microtubule inhibitor according to claim 4, wherein the taxane anticancer drug is at least one selected from the group consisting of cabazitaxel, paclitaxel, and docetaxel.

6. The therapeutic agent for cellular drug resistance to a microtubule inhibitor according to claim 4 or 5, wherein the taxane anticancer drug is cabazitaxel.

7. The therapeutic agent for cellular drug resistance to a microtubule inhibitor according to any one of claims 4 to 6, wherein the cancer is at least one selected from the group consisting of prostate cancer, bladder cancer, breast cancer, stomach cancer, and uterine cancer.

8. The therapeutic agent for cellular drug resistance to a microtubule inhibitor according to any one of claims 4 to 7, wherein the cancer is prostate cancer.

9. The therapeutic agent for cellular drug resistance to a microtubule inhibitor according to any one of claims 4 to 7, wherein the cancer is breast cancer.

10. The therapeutic agent for cellular drug resistance to a microtubule inhibitor according to any one of claims 4 to 7, wherein the cancer is uterine cancer.

11. A pharmaceutical composition for cancer treatment, the pharmaceutical composition comprising:

    a κ opioid receptor agonist; and
    a microtubule inhibitor.

12. The pharmaceutical composition for cancer treatment according to claim 11, wherein the κ opioid receptor agonist is at least one selected from the group consisting of spiradoline, nalfurafine, ICI-199, 441, LPK-26, BRL-52537, GR-89696, and salts thereof.

13. The pharmaceutical composition for cancer treatment according to claim 11 or 12, wherein the κ opioid receptor agonist is at least one of spiradoline and salts thereof.

14. The pharmaceutical composition for cancer treatment according to any one of claims 11 to 13, wherein the microtubule inhibitor is a taxane anticancer drug.

15. The pharmaceutical composition for cancer treatment according to claim 14, wherein the taxane anticancer drug is at least one selected from the group consisting of cabazitaxel, paclitaxel, and docetaxel.

16. The pharmaceutical composition for cancer treatment according to claim 14 or 15, wherein the taxane anticancer drug is cabazitaxel.

17. The pharmaceutical composition for cancer treatment according to any one of claims 11 to 16, wherein the cancer is at least one selected from the group consisting of prostate cancer, bladder cancer, breast cancer, stomach cancer, and uterine cancer.

**18.** The pharmaceutical composition for cancer treatment according to any one of claims 11 to 17, wherein the cancer is prostate cancer.

**19.** The pharmaceutical composition for cancer treatment according to any one of claims 11 to 17, wherein the cancer is breast cancer.

**20.** The pharmaceutical composition for cancer treatment according to any one of claims 11 to 17, wherein the cancer is uterine cancer.

# Fig. 1

| Combination compound |
|---|
| DMSO |
| 1 μmol/L Heliotrine |
| 1 μmol/L Albendazole |
| 300 nmol/L 6-bromoindirubin-3'-oxime (BIO) |
| 100 nmol/L Vorinostat |
| 1 μmol/L Esculetin |
| 3 μmol/L Sulfachloropyridazine |
| 1 μmol/L Metolazone |
| 10 μmol/L Troglitazone |
| 10 μmol/L Cotinine |
| 3 μmol/L Cefixime |
| 3 μmol/L 1-deoxy-16,16-dimethyl-prostaglandin E2 |
| 3 μmol/L 16,16-dimethyl-prostaglandin E2 |
| 10 μmol/L Spiradoline |
| 3 μmol/L Betazole |

Fig. 2

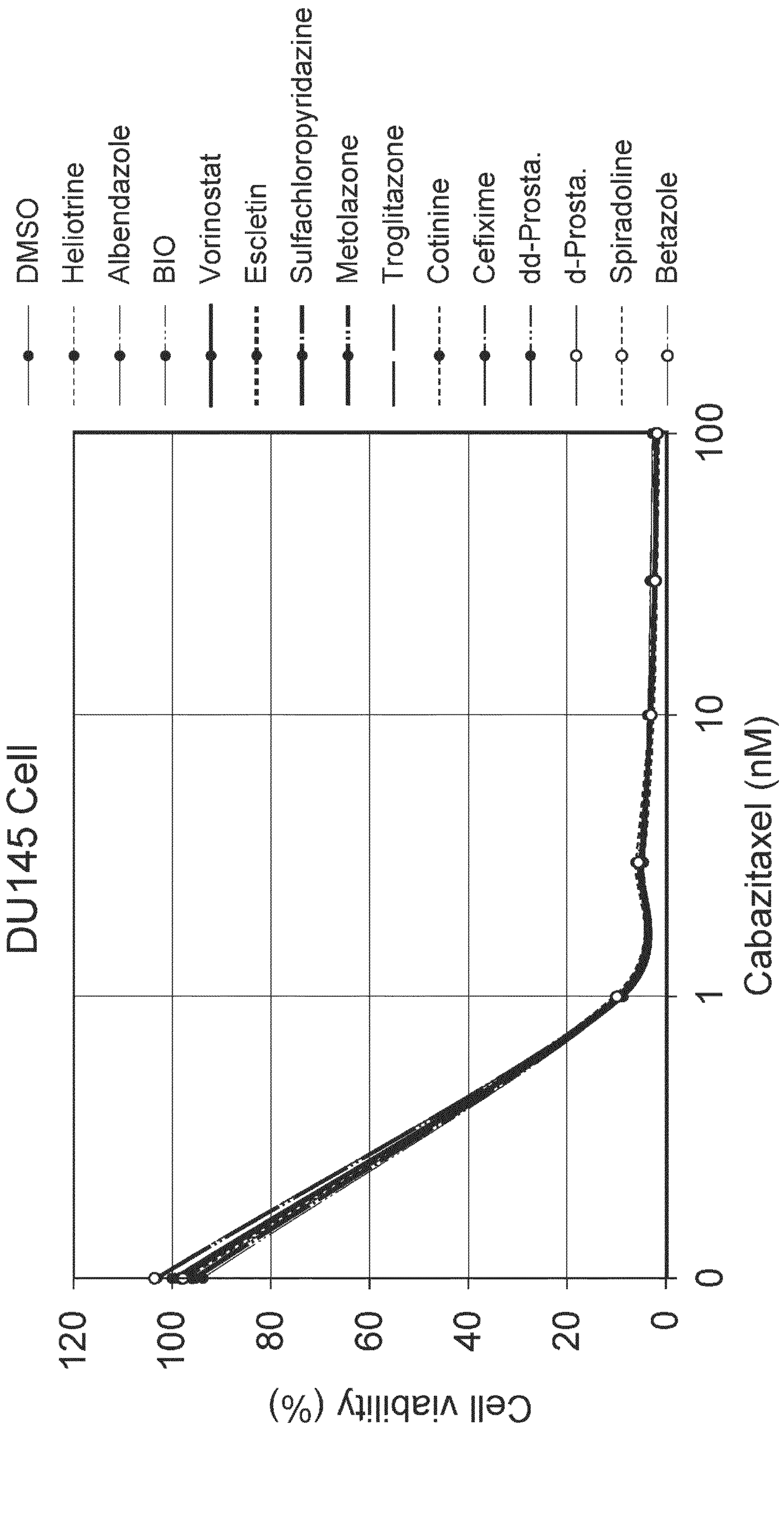
DU145 Cell
Cell viability (%)
120
100
80
60
40
20
0
0
1
10
100
Cabazitaxel (nM)
DMSO
Heliotrine
Albendazole
BIO
Vorinostat
Escletin
Sulfachloropyridazine
Metolazone
Troglitazone
Cotinine
Cefixime
dd-Prosta.
d-Prosta.
Spiradoline
Betazole

Fig. 3

DU145_TxR

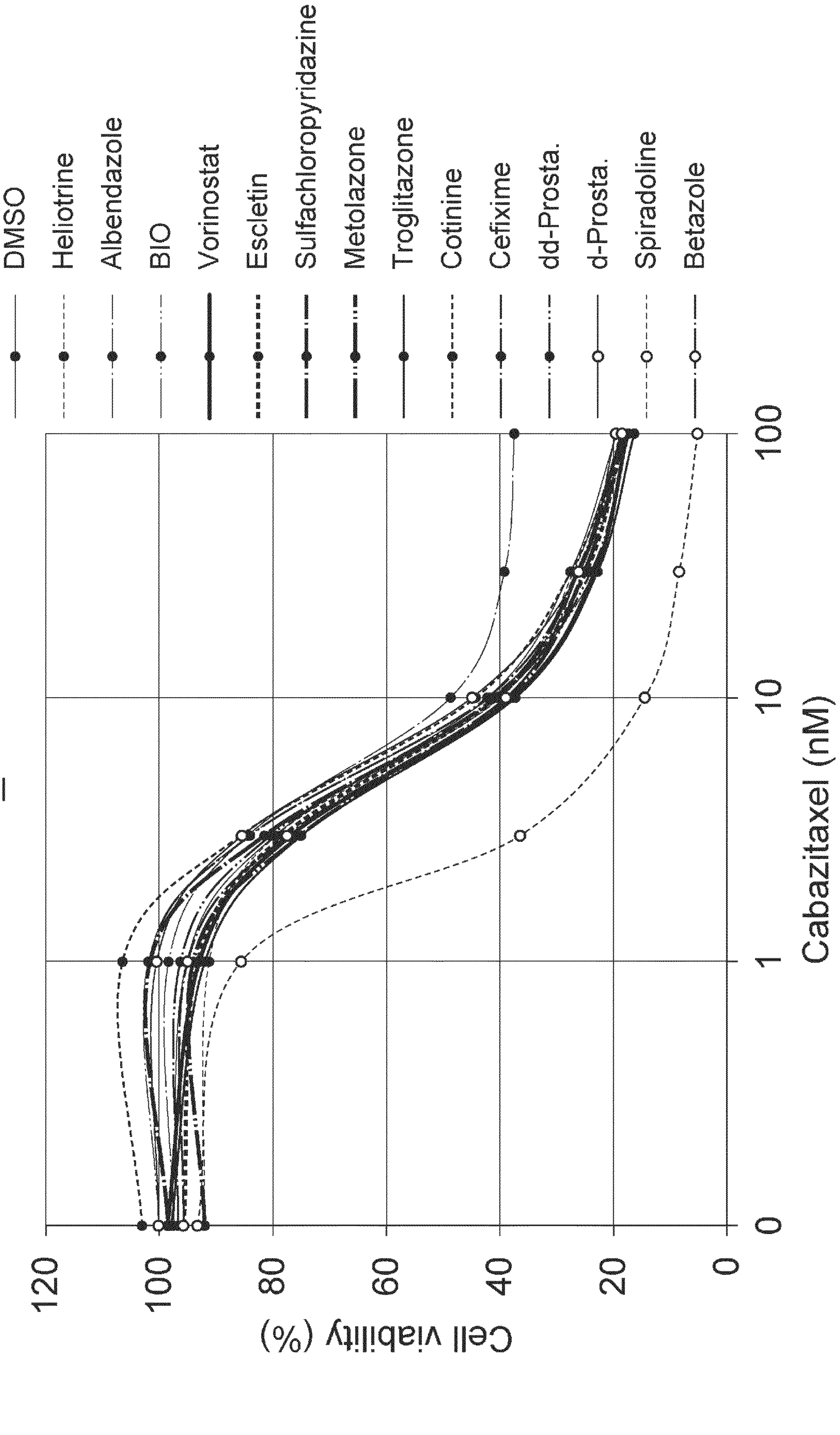

Fig. 4

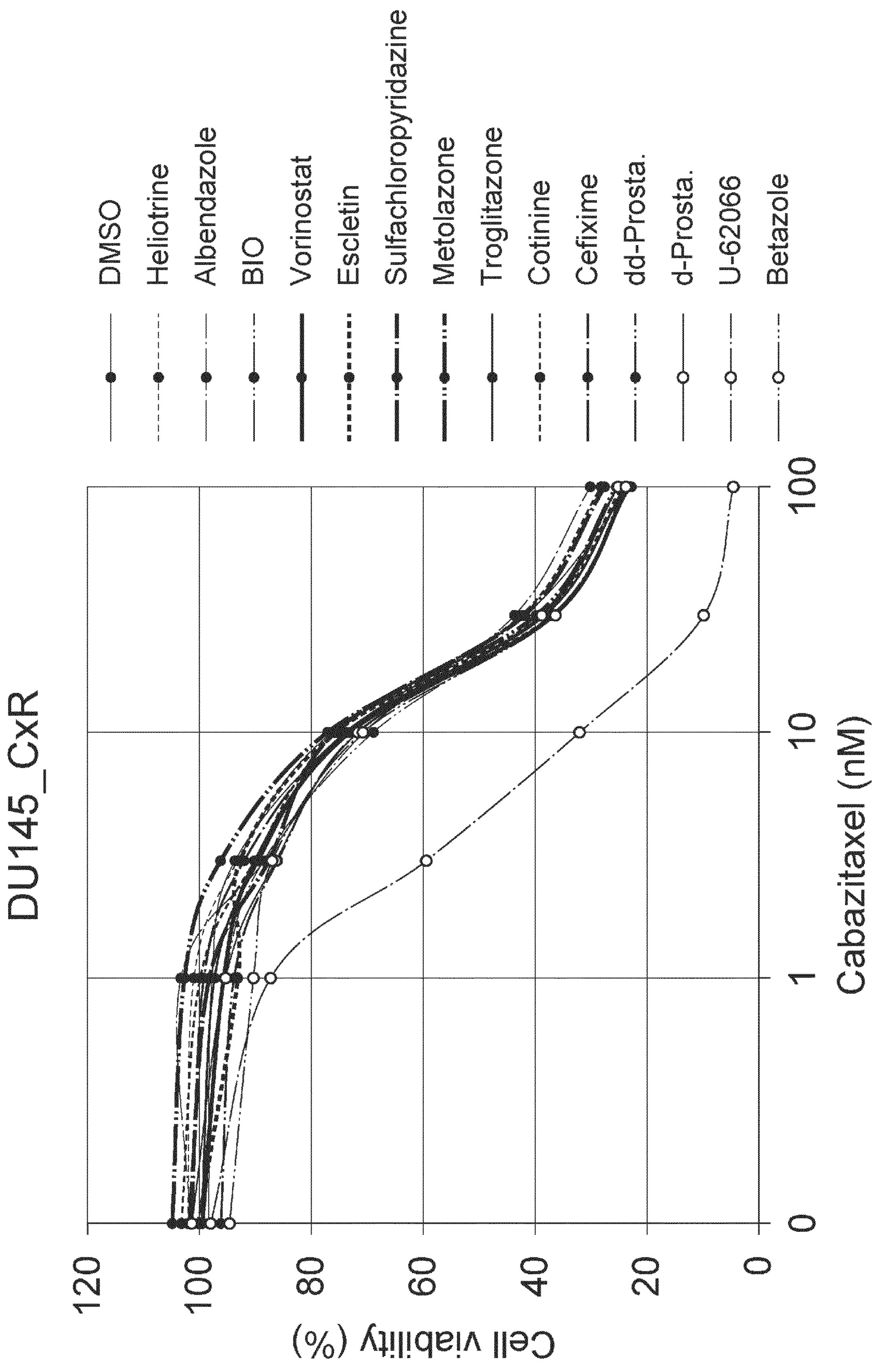
DU145_CxR
Cell viability (%)
120
100
80
60
40
20
0
Cabazitaxel (nM)
0
1
10
100
DMSO
Heliotrine
Albendazole
BIO
Vorinostat
Escletin
Sulfachloropyridazine
Metolazone
Troglitazone
Cotinine
Cefixime
dd-Prosta.
d-Prosta.
U-62066
Betazole

Fig. 5

$IC_{50}$ of Cabazitaxel (nmol/L)

| | DU145 | DU145_TxR | DU145_CxR |
|---|---|---|---|
| Combination use with DMSO | 0.36 | 7.77 | 22.66 |
| Combination use with Spiradoline | 0.35 | 2.21 | 4.54 |

Fig. 6

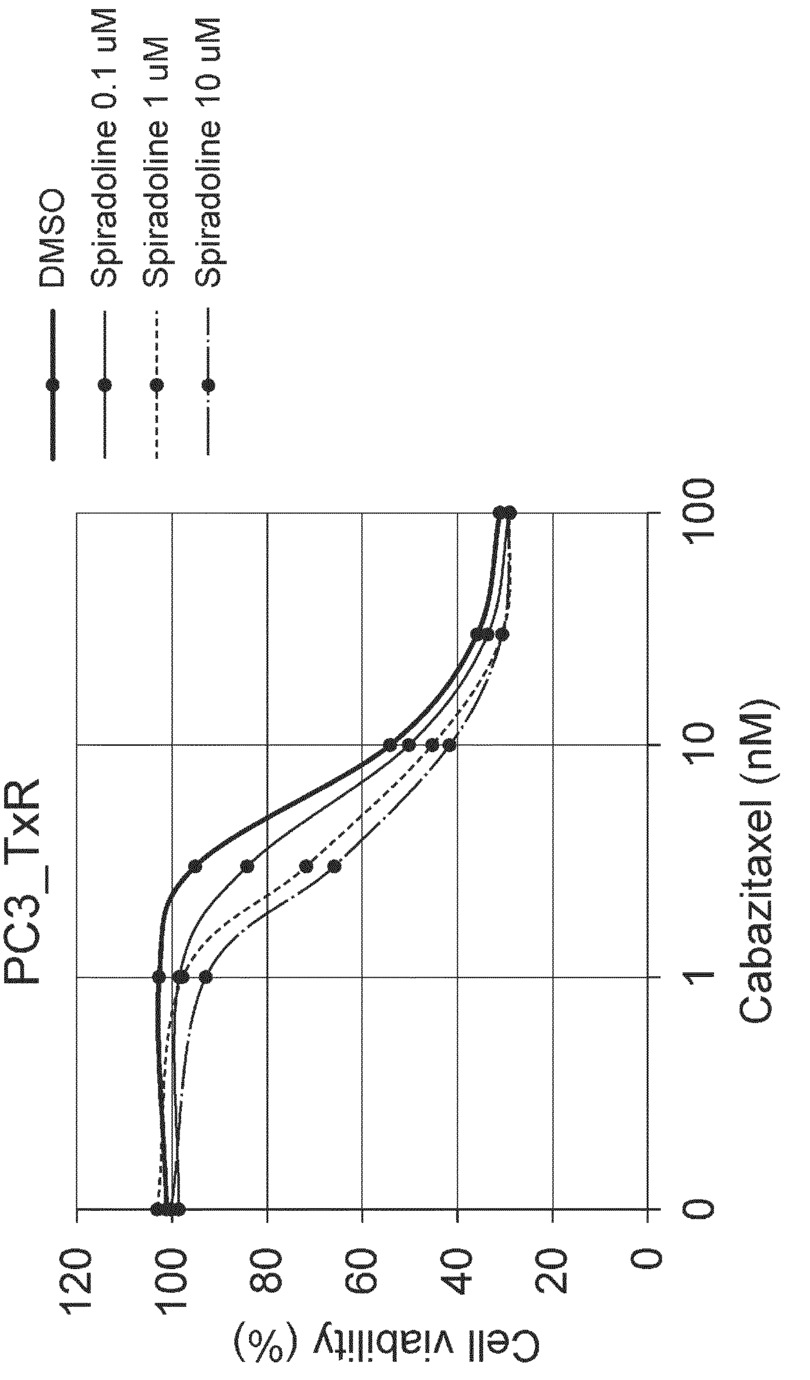
PC3_TxR
DMSO
Spiradoline 0.1 uM
Spiradoline 1 uM
Spiradoline 10 uM
Cell viability (%)
120
100
80
60
40
20
0
0
1
10
100
Cabazitaxel (nM)

Fig. 7

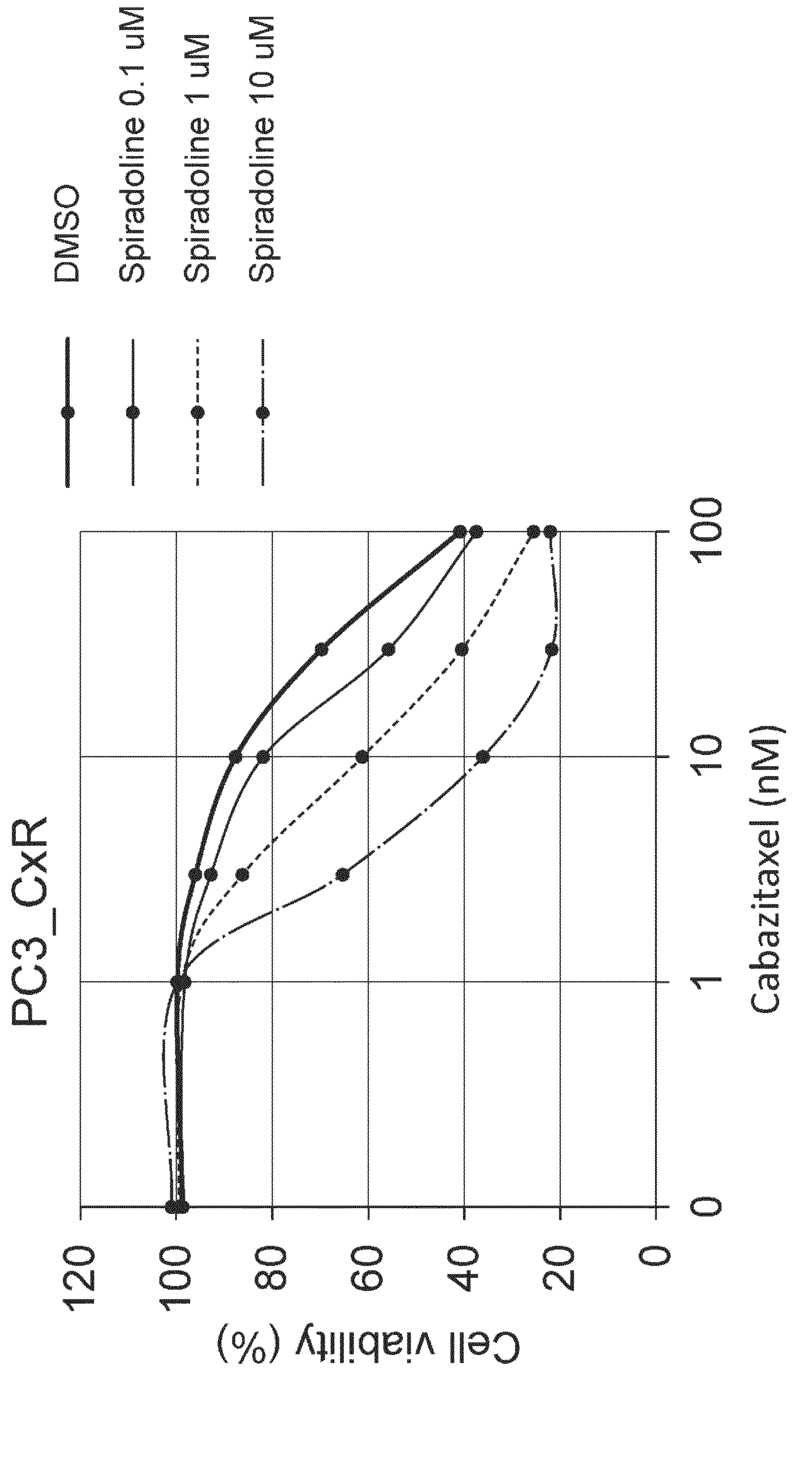
PC3_CxR
Cell viability (%)
120
100
80
60
40
20
0
0
1
10
100
Cabazitaxel (nM)
DMSO
Spiradoline 0.1 uM
Spiradoline 1 uM
Spiradoline 10 uM

Fig. 8

$IC_{50}$ of Cabazitaxel (nmol/L)

| | PC3_TxR | PC3_CxR |
|---|---|---|
| Combination use with DMSO | 12.8 | 68.3 |
| Combination use with 10 μmol/L Spiradoline | 6.6 | 5.6 |

Fig. 9

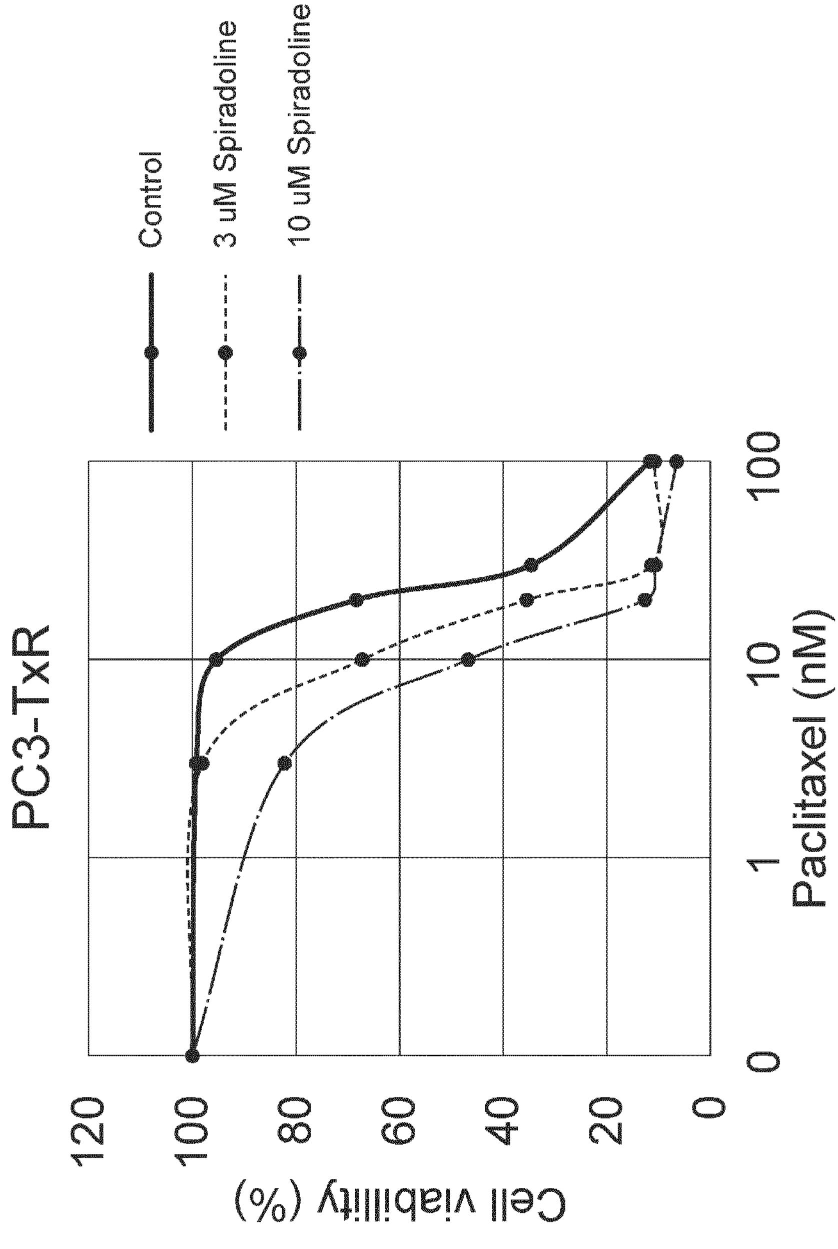
PC3-TxR
Control
3 uM Spiradoline
10 uM Spiradoline
Cell viability (%)
120
100
80
60
40
20
0
0
1
10
100
Paclitaxel (nM)

Fig. 10

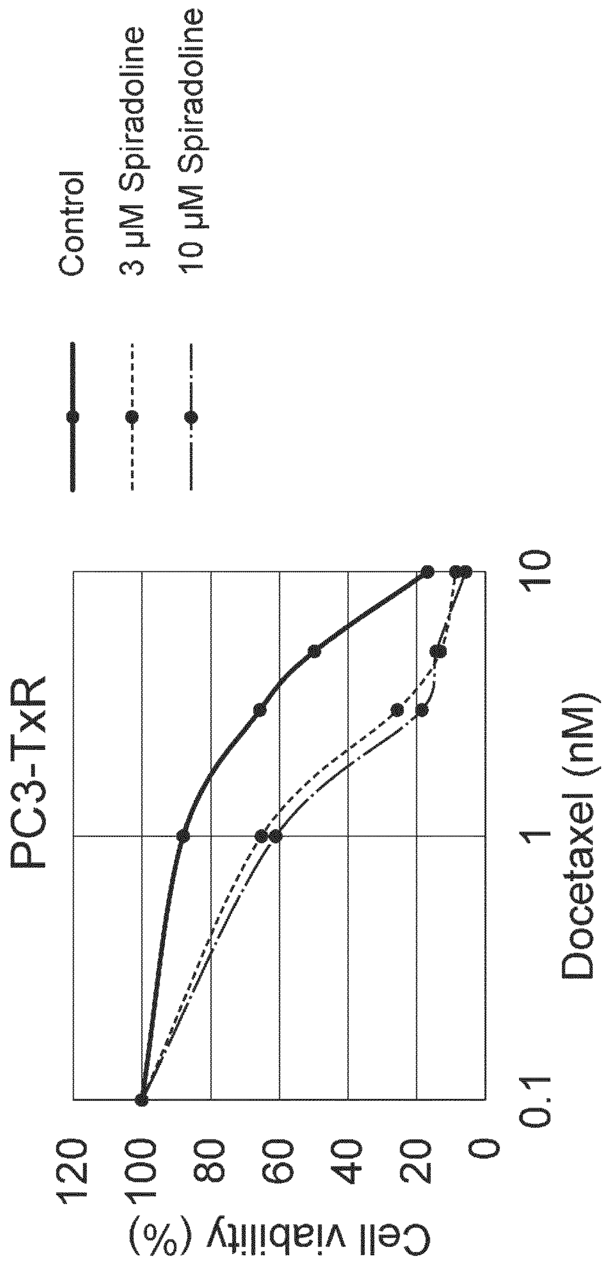
PC3-TxR
Cell viability (%)
120
100
80
60
40
20
0
0.1
1
10
Docetaxel (nM)
Control
3 μM Spiradoline
10 μM Spiradoline

# Fig. 11

$IC_{50}$ of Cabazitaxel with $\kappa$ opioid receptor agonist (nmol/L)

| Compounds | DU145_CxR | PC3_CxR |
|---|---|---|
| DMSO | 28.14 | 23.83 |
| Spiradoline | 6.01 | 4.91 |
| Nalfurafinee | 16.51 | 15.71 |
| ICI-199,441 | 4.13 | 3.63 |
| LPK-26 | 6.44 | 5.96 |
| BRL 52537 | 7.42 | 7.43 |
| GR 89696 | 8.53 | 6.94 |

Fig. 12

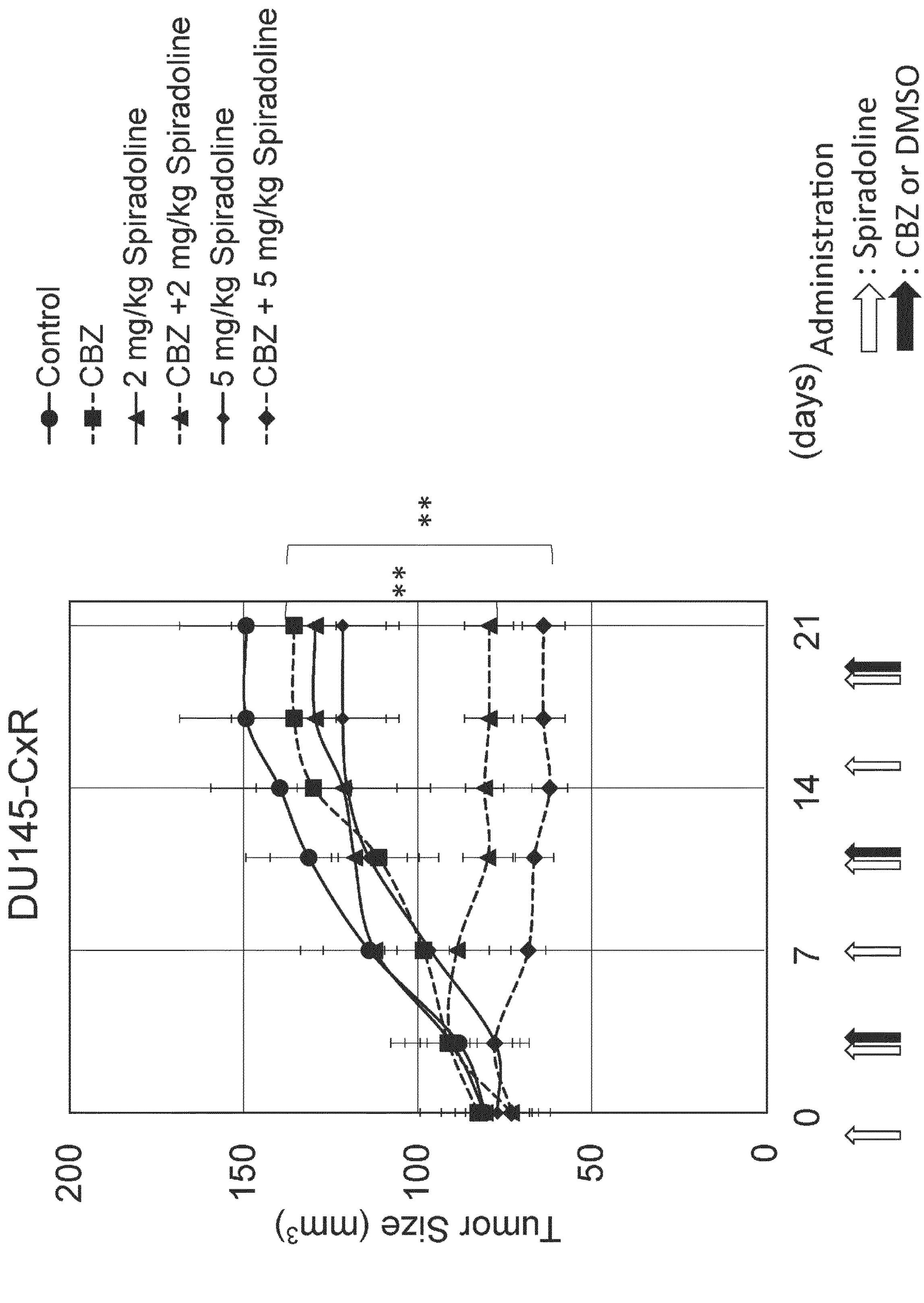
DU145-CxR
Tumor Size (mm³)
200
150
100
50
0
0
7
14
21
(days)
**
**
Control
CBZ
2 mg/kg Spiradoline
CBZ +2 mg/kg Spiradoline
5 mg/kg Spiradoline
CBZ + 5 mg/kg Spiradoline
Administration
: Spiradoline
: CBZ or DMSO

Fig. 13

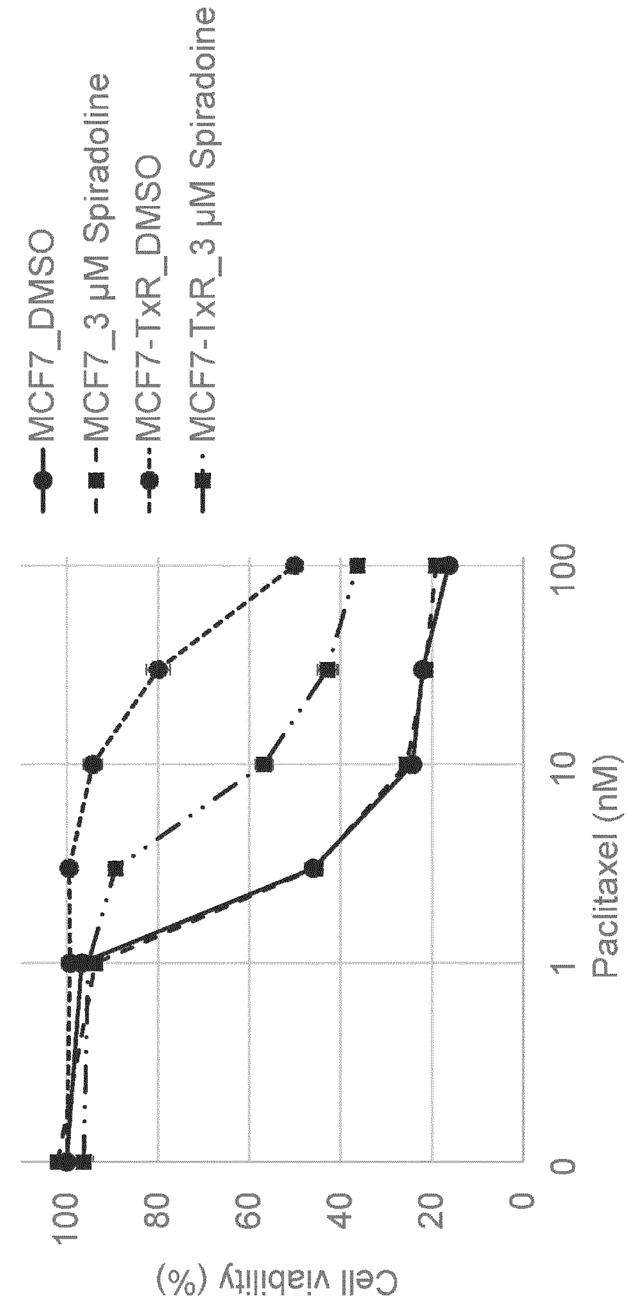
MCF7_DMSO
MCF7_3 µM Spiradoline
MCF7-TxR_DMSO
MCF7-TxR_3 µM Spiradoline
Cell viability (%)
100
80
60
40
20
0
0
1
10
100
Paclitaxel (nM)

Fig. 14

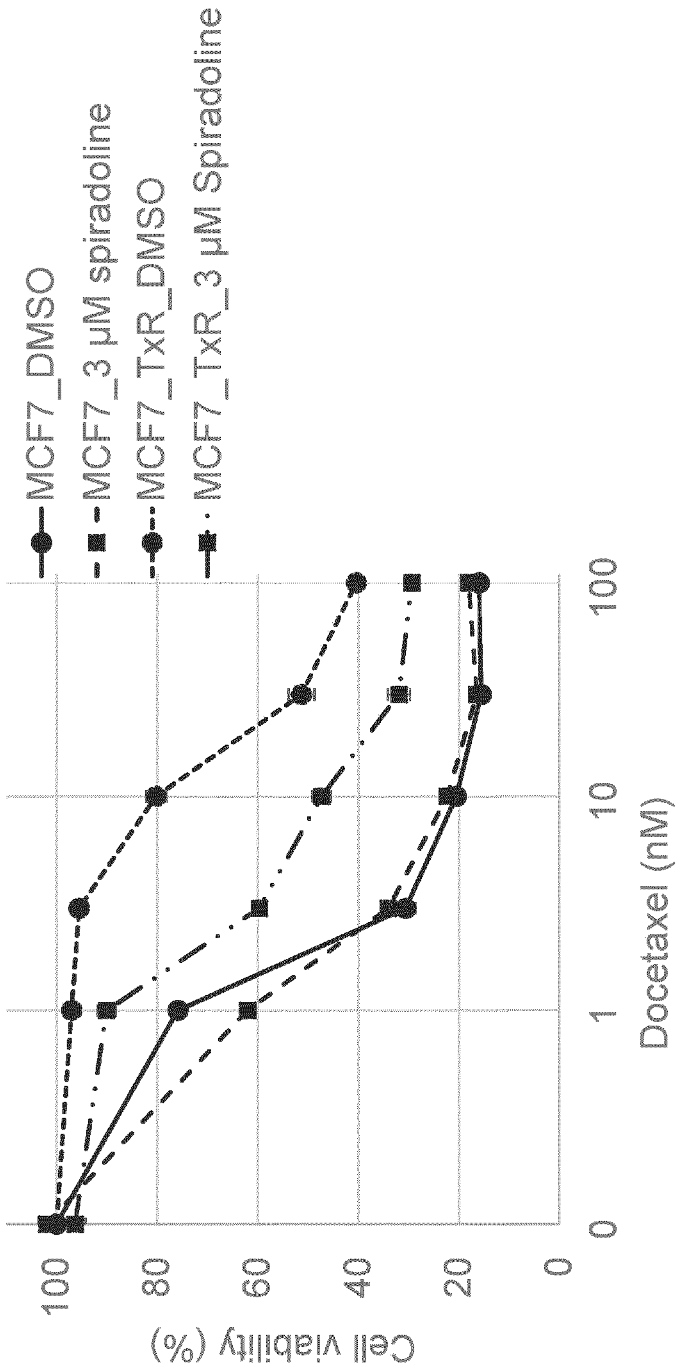
MCF7_DMSO
MCF7_3 µM spiradoline
MCF7_TxR_DMSO
MCF7_TxR_3 µM Spiradoline
Cell viability (%)
100
80
60
40
20
0
0
1
10
100
Docetaxel (nM)

Fig. 15

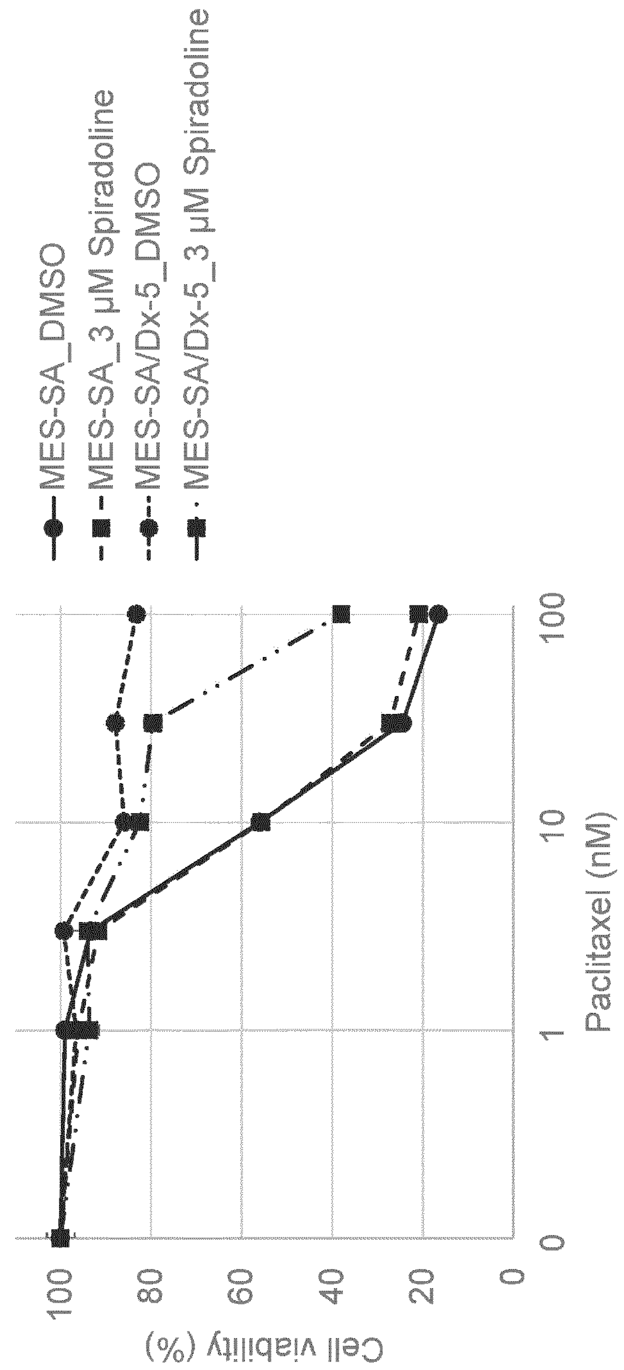
Cell viability (%)
100
80
60
40
20
0
0
1
10
100
Paclitaxel (nM)
MES-SA_DMSO
MES-SA_3 μM Spiradoline
MES-SA/Dx-5_DMSO
MES-SA/Dx-5_3 μM Spiradoline

Fig. 16

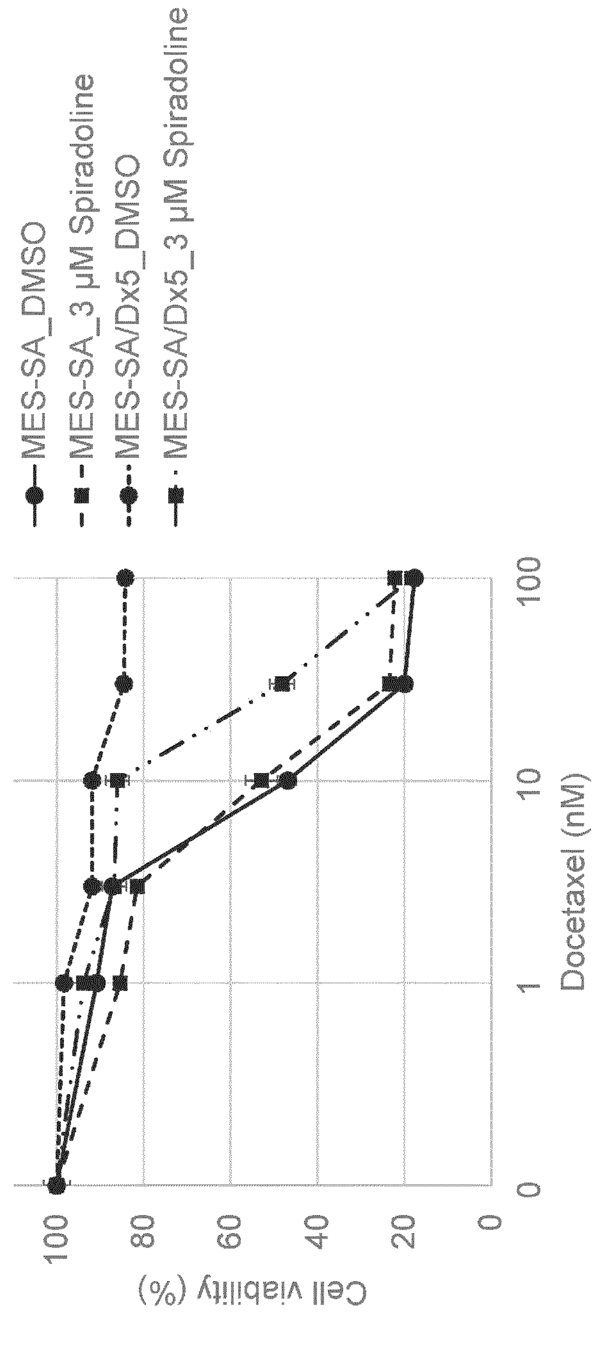
MES-SA_DMSO
MES-SA_3 µM Spiradoline
MES-SA/Dx5_DMSO
MES-SA/Dx5_3 µM Spiradoline
Cell viability (%)
100
80
60
40
20
0
Docetaxel (nM)
0
1
10
100

**INTERNATIONAL SEARCH REPORT**

International application No.
**PCT/JP2022/006631**

**A. CLASSIFICATION OF SUBJECT MATTER**

***A61K 45/00***(2006.01)i; ***A61P 35/00***(2006.01)i; ***A61P 43/00***(2006.01)i; ***A61K 31/335***(2006.01)i; ***A61K 31/40***(2006.01)i; ***A61K 31/4025***(2006.01)i; ***A61K 31/454***(2006.01)i; ***A61K 31/485***(2006.01)i; ***A61K 31/496***(2006.01)i
FI: A61K45/00; A61K31/335; A61P35/00; A61P43/00 105; A61P43/00 121; A61K31/4025; A61K31/485; A61K31/40; A61K31/454; A61K31/496

According to International Patent Classification (IPC) or to both national classification and IPC

**B. FIELDS SEARCHED**

Minimum documentation searched (classification system followed by classification symbols)

A61K45/00; A61P35/00; A61P43/00; A61K31/335; A61K31/40; A61K31/4025; A61K31/454; A61K31/485; A61K31/496

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | 日高隆雄. タキサン系薬剤. 薬局. 2011, vol. 62, no. 11, pp. 88-92, non-official translation (HIDAKA, Takao. Taxane-Based Drugs. Pharmacy.) p. 89, right column, paragraph 1 to p. 90, left column, paragraph 1 | 11, 14-20 |
| Y | | 12, 13 |
| A | | 1-10 |
| X | PATON, K. F. et al. The analgesic and anti-inflammatory effects of Salvinorin A analogue beta-tetrahydropyran Salvinorin B in mice. Eur. J. Pain. 2017, vol. 21, pp. 1039-1050 abstract | 11, 14-20 |
| Y | | 12, 13 |
| A | | 1-10 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 March 2022** | **05 April 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

INTERNATIONAL SEARCH REPORT

International application No.
PCT/JP2022/006631

C. DOCUMENTS CONSIDERED TO BE RELEVANT

| Category* | Citation of document, with indication, where appropriate, of the relevant passages | Relevant to claim No. |
|---|---|---|
| X | ATIGARI, Diana Vivian et al. The mixed kappa and delta opioid receptor agonist, MP1104, attenuates chemotherapy-induced neuropathic pain. Neuropharmacology. 2021, vol. 185, 108445, Available online, 28 December 2020<br>abstract | 11, 14-20 |
| Y | | 12, 13 |
| A | | 1-10 |
| X | JP 2011-512338 A (LABORATORIOS DEL DR ESTEVE SA) 21 April 2011 (2011-04-21)<br>claims, examples | 11, 12, 14-20 |
| A | | 1-10, 13 |
| Y | JP 2020-516591 A (JIANGSU HENGRUI MEDICINE CO., LTD.) 11 June 2020 (2020-06-11)<br>claim 8 | 12, 13 |

Form PCT/ISA/210 (second sheet) (January 2015)

**INTERNATIONAL SEARCH REPORT**
**Information on patent family members**

International application No.
**PCT/JP2022/006631**

| Patent document cited in search report | Publication date (day/month/year) | Patent family member(s) | Publication date (day/month/year) |
|---|---|---|---|
| JP 2011-512338 A | 21 April 2011 | WO 2009/103487 A1<br>claims, examples<br>US 2017/0112829 A1<br>US 2011/0052723 A1<br>EP 2090311 A1<br>EP 2254579 A1<br>CN 101951908 A<br>KR 10-2011-0008166 A | |
| JP 2020-516591 A | 11 June 2020 | US 2020/0054594 A1<br>claim 24<br>WO 2018/188641 A1<br>EP 3610874 A1<br>CN 109982699 A<br>KR 10-2019-0133047 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 6735484 B


### Non-patent literature cited in the description


- *CHEMICAL ABSTRACTS,* 87151-85-7
- *CHEMICAL ABSTRACTS,* 152658-17-8
- *CHEMICAL ABSTRACTS,* 115199-84-3
- *CHEMICAL ABSTRACTS,* 492451-07-7
- *CHEMICAL ABSTRACTS,* 130497-33-5
- *CHEMICAL ABSTRACTS,* 126766-32-3
- *Prostate,* 2007, vol. 67 (9), 955-967
- *Cancer Res.,* 1985, vol. 45, 4091-4096
- **CHEN G ; DURAN GE ; STEGER KA ; LACAYO NJ ; JAFFREZOU JP ; DUMONTET C ; SIKIC BI.** Multidrug-resistant human sarcoma cells with a mutant P-glycoprotein, altered phenotype, and resistance to cyclosporins. *J Biol Chem.,* 1997, vol. 272, 5974-5982